# EUROPEAN PATENT APPLICATION

(11) **EP 2 009 445 A1**
(43) Date of publication of application: **31.12.2008**
(21) Application number: 07290812.2
(22) Date of filing: 29.06.2007
(51) Int. Cl.: G01N 33/68, C07K 14/47, C07K 16/18

(54) **Use of a camelid single-domain antibody for detecting an oligomeric form of an amyloid beta peptide and its applications**

(71) Applicant: Institut Pasteur, 75015 Paris (FR); Centre National de la Recherche Scientifique, 75016 Paris (FR)
(72) Inventor: Rougeon, François, 92310 Sèvres (FR); Lafaye, Pierre, 92240 Malakoff (FR)
(74) Representative: Vialle-Presles, Marie José

(57) **Abstract**

Use of camelid single-domain antibodies for detecting an oligomeric form of the amyloid β peptide 42 and their therapeutic and diagnostic applications.

SEQUENCE LISTING

<110>  INSTITUT PASTEUR
       CENTRE NATIONAL DE LA RECHERCHE SCIENTIFIQUE

<120>  Use of camelid single-domain antibodies for detecting an
       oligomeric form of an amyloid beta peptide and their applications

<130>  F226EP143

<160>  12

<170>  PatentIn version 3.3

<210>  1
<211>  122
<212>  PRT
<213>  Camelidae

<400>  1

Ala Glu Val Gln Leu Gln Ala Ser Gly Gly Gly Ser Val Gln Pro Gly
1               5                   10                  15

Gly Ser Leu Arg Leu Ser Cys Ala Ala Ser Gly Phe Ile Phe Gly Trp
            20                  25                  30

Ser Thr Met Ser Trp Val Arg Gln Ala Pro Gly Lys Gly Leu Glu Trp
        35                  40                  45

Val Ser Thr Ile Ser Gly Gly Gly Ser Ala Thr Thr Tyr Thr Asp Ser
    50                  55                  60

Val Lys Gly Arg Phe Thr Ile Ser Arg Asp Arg Ala Lys Asn Thr Leu
65                  70                  75                  80

Tyr Leu Gln Met Asn Ser Leu Lys Pro Glu Asp Thr Ala Ile Tyr Tyr
                85                  90                  95

Cys Asn Ala Asp Val Ser Thr Gly Phe Arg Tyr Gln Arg Lys Asp Tyr
            100                 105                 110

Trp Gly Arg Gly Thr Gln Val Thr Val Ser
        115                 120

<210>  2
<211>  42
<212>  PRT
<213>  Artificial

<220>
<223>  Description de la séquence artificielle: Peptide synthétique

<400>  2

<210>  3
<211>  118
<212>  PRT
<213>  Camelidae

<400>  3

Ala Glu Val Gln Leu Gln Ala Ser Gly Gly Gly Leu Val Gln Ala Gly
1               5                   10                  15

Gly Ser Leu Arg Leu Ser Cys Ser Ala Ser Ala Ser Thr Thr Phe Ser
            20                  25                  30

Met Asn Thr Met Ala Trp His Arg Gln Ala Pro Gly Lys Gln Arg Ser
        35                  40                  45

Leu Val Ala Leu Ile Gly Ala Thr His Ser Ile Asn Tyr Glu Asp Ser
    50                  55                  60

Val Lys Gly Arg Phe Thr Ile Ser Arg Asp Asn Ala Lys Asn Thr Val
65                  70                  75                  80

Tyr Leu Gln Met Ser Ser Leu Lys Pro Glu Asp Thr Ala Val Tyr Tyr
                85                  90                  95

Cys Asn Asp Trp Tyr Trp Gln Met Lys Gly Gly Ser Trp Gly Gln Gly
            100                 105                 110

Thr Gln Val Thr Val Ser
        115

<210>  4
<211>  116
<212>  PRT
<213>  Camelidae

<400>  4

Ala Glu Val Gln Leu Gln Ala Ser Gly Gly Gly Leu Val Gln Ala Gly
1               5                   10                  15

Gly Ser Leu Arg Leu Ser Cys Ala Ala Ser Gly Ser Thr Phe Ser Ile
            20                  25                  30

Asn Val Ile Gly Trp Tyr Arg Gln Ala Pro Gly Lys Gln Arg Glu Leu
        35                  40                  45

Val Ala Gly Ile Ser Arg Ser Gly Asn Thr Asn Tyr Ala Asp Ser Val
    50                  55                  60

Glu Gly Arg Phe Thr Ile Ser Arg Asp Tyr Ala Lys Asn Thr Val Tyr
65                  70                  75                  80

Leu Gln Met Asn Ser Leu Lys Pro Glu Asp Thr Ala Val Tyr Tyr Cys
                85                  90                  95

His Ser Lys Thr Tyr Leu Leu His Met Tyr Trp Gly Gln Gly Thr Gln
            100                 105                 110

Val Thr Val Ser
        115

<210>  5
<211>  11
<212>  PRT
<213>  Artificial

<220>
<223>  Peptide synthétique

<400>  5

Asp Ala Glu Phe Arg His Asp Ser Gly Tyr Glu
1               5                   10

<210>  6
<211>  11
<212>  PRT
<213>  Artificial

<220>
<223>  Peptide synthétique

<400>  6

Tyr Glu Val His His Gln Lys Leu Val Phe Phe
1               5                   10

<210>  7
<211>  11
<212>  PRT
<213>  Artificial

<220>
<223>  Peptide synthétique

<400>  7

Gln Lys Leu Val Phe Phe Ala Glu Asp Val Gly
1               5                   10

<210>  8
<211>  14
<212>  PRT
<213>  Artificial

<220>
<223>  Peptide synthétique

<400>  8

Glu Asp Val Gly Ser Asn Lys Gly Ala Ile Ile Gly Leu Met
1               5                   10

<210>  9
<211>  12
<212>  PRT
<213>  Artificial

<220>
<223>  Peptide synthétique

<400>  9

Gly Ala Ile Ile Gly Leu Met Val Gly Gly Val Val
1               5                   10

<210>  10
<211>  10
<212>  PRT
<213>  Artificial

<220>
<223>  Peptide synthétique

<400>  10

Gly Leu Met Val Gly Gly Val Val Ile Ala
1               5                   10

<210>  11
<211>  8
<212>  PRT
<213>  Artificial

<220>
<223>  Peptide synthétique

<400>  11

Met Val Gly Gly Val Val Ile Ala
1               5

<210>  12
<211>  114
<212>  PRT
<213>  Camelidae

<400>  12

Ala Glu Val Gln Leu Gln Ala Ser Gly Gly Gly Leu Val Gln Ala Gly
1               5                   10                  15

Gly Ser Leu Arg Leu Ser Cys Ala Ala Ser Gly Ser Thr Phe Arg Ile
            20                  25                  30

Asn Arg Met Gly Trp Tyr Arg Gln Ala Pro Gly Lys Gln Arg Glu Leu
        35                  40                  45

Val Ala Ser Ile Asn Ser Gly Gly Ser Thr Asn Tyr Ala Asp Ser Val
    50                  55                  60

Lys Gly Arg Phe Thr Ile Ser Arg Asp Asn Ala Lys Gly Thr Val Asn
65                  70                  75                  80

Leu Thr Met Asn Ser Leu Lys Pro Glu Asp Thr Ala Val Tyr Tyr Cys
                85                  90                  95

Asn Arg Val Thr Pro Trp Pro Tyr Trp Gly Gln Gly Thr Gln Val Thr
            100                 105                 110

Val Ser

## Description

The invention relates to the use of camelid single-domain antibodies for detecting an oligomeric form of the amyloid β peptide 42 and their applications.

Alzheimer's disease (AD) is a progressive, irreversible brain disorder with no known cause or cure. Extracellular fibrillar deposits and senile plaques are prominent and universal AD features. The major component of these aggregates is a 40 or 42 amino acid polypeptide called Amyloid β (Aβ). However, the initial focus on the fibrillar amyloid as the central structure in AD pathology has evolved during the last 10 years. This was due to several outstanding discoveries such as the finding of a soluble fraction of oligomeric Aβ in the human brain (Kuo *et al.,* 1996). These isolated soluble oligomers were toxic to neurons in culture. The presence and toxicity of oligomeric Aβ was then confirmed and the name of ADDLs (Aβ-derived diffusible ligands) was proposed for these structures (Lambert *et al.,* 1998). Depending on conditions, ADDL compositions can contain predominantly trimers-hexamers, with larger structures of up to 24-mers. ADDLs show important regionally selective neurotoxicity, sparing neurons in the cerebellum while selectively killing neurons in hippocampal CA1 region and entorhinal cortex (Klein *et al.,* 2001). Moreover, oligomers are able to inhibit hippocampal long-term potentiation (LTP) in rats *in vivo* (Walsh *et al.,* 2002) and in hippocampal slices (Wang *et al.,* 2002; Wang *et al.,* 2004). Recently, it has been showed that cognitive deficits are directly attributable to low amounts of soluble oligomeric forms of Amyloid β; trimers and at a lesser extent, dimers and tetramers being particularly active (Cleary *et al.,* 2005; Townsend *et al.,* 2006).

Different models have been proposed for the formation of Aβ fibrils. A simple model proposed by Bitan *et al.* (2003) illustrates how Aβ42 may assemble. Monomers rapidly oligomerize into paranuclei that in turn associate to form large oligomers and protofibrils. Monomers, paranuclei (pentamers, hexamers), and oligomers are predominantly unstructured, but do contain some β-strand and α-helix elements. Protofibril formation involves substantial conformational rearrangements, during which unstructured, α-helix and β-strand elements transform into predominantly β sheet/β-turn structures. The final step in the pathway is protofibril maturation into fibrils, a process that appears to be irreversible, at least kinetically (Lomakin *et al.,* 1997).

The need for accurate diagnosis of disorder mediated by amyloid β peptide oligomers such as AD or the Down syndrome is increasingly important as therapeutics become available in some cases. Therefore, a reliable assay for Aβ oligomers would be extremely helpful. However these assays are impaired because monoclonal and polyclonal antibodies, generated by immunization with Aβ oligomers, raised against oligomers also recognize fibrils (Lacor *et al.,* 2004; Lambert *et al.,* 2001; Lambert *et al.,* 2007). These antibodies show, in AD brain sections, the expected AD type immunoreactivity and also a pericellular immunoreactivity (Lambert *et al.,* 2007; Lacor *et al.,* 2004; Gong *et al.,* 2003). A monoclonal antibody raised against oligomers was also obtained by Lee *et al.* (2006). It recognizes oligomers, fibrils and mature senile plaques on AD brain sections. Kayed *et al.* (2003) prepared polyclonal rabbit antibodies that specifically recognize a conformation specific of Aβ high-MW oligomers distinct from that of soluble monomers, low-MW oligomers, and fibrils. A way to discriminate between the different conformations of Aβ is to use alternative "binders" which could recognize non conventional epitopes.

A significant proportion of camelid antibodies are single-domain antibodies, which interact with the antigen via a single heavy-chain binding domain devoid of light chain. These antibodies are referred to as "VHH" or "VHH antibodies". Recombinant VHH is the minimal-sized, intact antigen-binding domain. The absence of VL domain allows the VHHs to attain a higher structural flexibility than that of VH domains associated with VLs. Furthermore, the complementarity determining regions (CDRs) of VHHs, and especially CDR3, are statistically longer than those of conventional VH-VL antibodies (Muyldermans *et al.,* 2001). Small size and increased plasticity appear to endow VHHs with unique potentialities: for instance, several VHHs are capable of inhibiting enzymatic activity by interacting with the active site cavity of enzymes such as α-amylase, carbonic anhydrase and hen egg lysozyme (Desmyter *et al.,* 1996; Desmyter *et al.,* 2002; Transue *et al.,* 1998; Lauwereys *et al.,* 1998). These features may allow camelid VHHs to recognize other unique epitopes that are poorly immunogenic for conventional antibodies.

International Application No. WO 2004/044204 describes variable fragments of camelid single-chain antibodies (VHH antibodies) capable to specifically bind the amyloid β peptide 42. Among these VHH antibodies, one particular antibody, referred to as VHH V31-1, has been shown to specifically recognize the carboxy terminal end of Aβ42 peptide (Aβ42) in its fibrillar form and intraneuronal Aβ42 deposits.

The Inventors have now found that contrary to what was described in said International Application No. WO 2004/044204, VHH V31-1 antibody does not recognize Aβ42 in its fibrillar form but specifically recognizes low-molecular oligomers of Aβ42.

Furthermore the Inventors have also found that unexpectedly said VHH V31-1 antibody inhibits fibrillogenesis, i.e. Aβ fibril formation, in spite of the fact that it specifically binds the C-terminus of Aβ42. Indeed, previous studies have indicated that antibodies specific of regions 1-6 (Solomon, 2002; Legleiter *et al.,* 2004) and 17-20 (Liu *et al.,* 2004; Legleiter *et al.,* 2004) of Aβ42 inhibited *in vitro* aggregation and cytotoxicity of Aβ, while antibodies specific of the C-terminus did not inhibit aggregation (Bard *et al.,* 2000; Liu *et al.,* 2004). The regions 17-20 and the C-terminus play critical roles in the aggregation (Ma and Nussinov, 2002; Balbach *et al.,* 2000; Halverson *et al.,* 1990; Jarrett *et al.,* 1993 and Hilbich *et al.,* 1991), which may explain why antibodies specific of the central region of Aβ inhibited aggregation. However there is no clear explanation why previously reported C-terminus specific antibodies had no effect on the aggregation of Aβ.

Therefore, in a first aspect, the present invention relates to the use of a VHH antibody of camelid binding an epitope located at the C-terminal end of the amyloid β peptide 42, for detecting *in vitro* in an appropriate biological sample or tissue, or *in vivo* in an organ (for instance the brain), an oligomeric form of the amyloid β peptide 42.

A VHH antibody of camelid (camel, dromedary, llama, alpaca,...) refers usually to a variable fragment of a camelid single-chain antibody (See Nguyen *et al.,* 2001; Muyldermans, 2001), and also comprises according to the present invention:
- an isolated VHH antibody of camelid,
- a recombinant VHH antibody of camelid, or
- a synthetic VHH antibody of camelid.

In a preferred embodiment, the VHH antibody of the present invention is a variable fragment of a camelid single-chain antibody having the amino acid sequence of SEQ ID NO: 1. This VHH antibody has been described in International Application No. WO 2004/044204. A host cell expressing VHH V31-1 is available at the Collection Nationale de Cultures de Microorganismes (CNCM), 28 rue du Dr. Roux, 75724 Paris Cedex 15, France, under the number 1-2936.

As used herein, an "amyloid β peptide" refers to a peptide generated from amyloid precursor protein (APP) by β- and γ-secretase-mediated cleavage. Preferably, the amyloid β peptide is the amyloid β peptide 42 (Aβ42) having the amino acid sequence of SEQ ID NO: 2.

As used herein, the term "oligomer" refers typically to the initially formed, metastable multimer in an amyloid formation reaction (Kodali *et al.,* 2007). Some protofibrils might also be considered oligomers.

In a preferred embodiment of the invention, the oligomeric form (oligomer) of said amyloid β peptide 42 is selected from the group consisting of a 2-mer, 3-mer, 4-mer, 5-mer, 6-mer, 7-mer, 8-mer, 9-mer, 10-mer, 11-mer, 12-mer, 13-mer, 14-mer, 15-mer, 16-mer, 17-mer, 18-mer, 19-mer, 20-mer, 21-mer, 22-mer, 23-mer, 24-mer, more preferably a dimer, trimer, tetramer or dodecamer (12-mer).

The characterization of the binding of a camelid VHH antibody according to the present invention to an epitope located at the C-terminal end of the amyloid β peptide 42 can be performed by an ELISA-based binding assay as described in Example 2 below. Advantageously, the dissociation constant of said VHH antibody is about or less than 2. 10⁻⁸ M. Dissociation constant measurements may be made using methods known to those skilled in the art, including using the method described in Friguet *et al.* (1985).

The term "C-terminal end of the amyloid β peptide 42" as used herein, refers to the amino acids 25 to 42, particularly to the amino acids 29 to 42 of the amyloid β peptide of SEQ ID NO:2.

In a preferred embodiment of the invention, the epitope comprises, is comprised or consists of a peptide selected from the group consisting of the peptide Aβ 29-40 having the amino acid sequence of SEQ ID NO: 9, the peptide Aβ 33-42 having the amino acid sequence of SEQ ID NO: 10 and the peptide Aβ 35-42 having the amino acid sequence of SEQ ID NO: 11 in the annexed listing of sequences.

The term "detecting" means assessing the presence or absence of an oligomeric form of the amyloid β peptide 42 as defined here above, *in vitro* or *ex vivo* in an appropriate biological sample or tissue (obtained for example by brain biopsy) or *in vivo* in an organ, for instance the brain. This process involves the ability of said VHH antibody (of SEQ ID NO: 1) to bind an oligomeric form of the amyloid β peptide 42.

The detection can be performed by any method known in the art *in vitro* in an appropriate biological sample or tissue or *in vivo* in an organ (for instance the brain). These methods include an appropriate immunochemical technique such as:
- ELISA, EIA, RIA, immunofluorescence, immunocytochemical, immunohistochemical,
- immunoblot analysis performed in a biological sample or tissue (e.g., blood, serum, urine, cerebrospinal fluid) from a subject,
- and also brain imaging.

By way of example, this may be accomplished by linking to said VHH antibody a detectable label that can be visualized or measured, or by using ligands (e.g., an antibody linked to a detectable label) that specifically bind to said VHH antibody.

The level of binding is preferably detected quantitatively.

Advantageously, the detection of the presence of an oligomeric form of the amyloid β peptide 42 as described above is a prognostic marker of appearance of a disorder mediated by amyloid β peptide oligomers, preferably a neurodegenerative disorder such as Alzheimer's disease or the Down syndrome.

In a preferred embodiment of the invention, the detectable label is selected from the group consisting of:
- an enzyme, such as horseradish peroxidise, alkaline phosphatase, glucose-6-phosphatase, beta-galactosidase;
- a fluorophore, such as green fluorescent protein (GFP), blue fluorescent dyes excited at wavelengths in the Itraviolet (UV) part of the spectrum (e.g. AMCA (7-amino-4-methylcoumarin-3-acetic acid); Alexa Fluor 350), green fluorescent dyes excited by blue light (e.g. FITC, Cy2, Alexa Fluor 488), red fluorescent dyes excited by green light (e.g. rhodamines, Texas Red, Cy3, Alexa Fluor dyes 546, 564 and 594), or dyes excited with far-red light (e.g. Cy5) to be visualized with electronic detectors (CCD cameras, photomultipliers);
- a heavy metal chelate, such as europium, lanthanum or yttrium;
- a radioisotope, such as [¹⁸F]fluorodeoxyglucose or ¹¹C-, ¹²⁵I-, ¹³¹I-, ³H-, ¹⁴C-, ⁹⁹Tc- and ³⁵S- labelled compounds.
- a biotin that can be detected using labeled avidin.

In a second aspect, the present invention provides a method for determining *in vitro* in an appropriate biological sample or a tissue, or *in vivo* in an organ (for instance the brain), an early-stage of a disorder mediated by amyloid β peptide oligomers, preferably by Aβ42 oligomers, (for instance a neurodegenerative disorder such as Alzheimer's disease or the Down Syndrome), comprising a step of detecting, in a subject (a mammal, preferably a human), an oligomeric form of an amyloid β peptide 42 as defined here above, by contacting said appropriate biological sample, tissue or organ with a VHH antibody as defined above, preferably the VHH antibody of SEQ ID NO: 1, preferably labelled with a detectable label as defined above.

According to another aspect, the present invention relates to a method of diagnostic of a disorder mediated by amyloid β peptide oligomers, preferably by Aβ42 oligomers as defined above, in a subject (a mammal, preferably a human) comprising the steps of:
a) contacting an appropriate biological sample, tissue or organ, with a VHH antibody of camelid binding an epitope located at the C-terminal end of an amyloid β peptide 42 as defined above, preferably the VHH antibody having the amino acid sequence of SEQ ID NO: 1, and
b) detecting the binding of said VHH antibody to said biological sample, tissue or organ, a binding constituting a marker of the presence of said disorder (for instance Alzheimer's disease or the Down Syndrome).

According to the invention, said contacting step may be performed *in vitro, ex vivo* or *in vivo.*

When the contacting step is performed *in vivo,* the organ is preferably the brain.

The present invention also relates to a method of diagnostic of a disorder mediated by amyloid β peptide oligomers, preferably by Aβ42 oligomers, in a subject (a mammal, preferably a human) comprising the steps of:
a) contacting *in vitro* or *ex vivo* an appropriate biological sample or tissue, or *in vivo* (for instance the brain), with a VHH antibody of camelid binding an epitope located at the C-terminal end of an amyloid β peptide 42 as defined above, preferably the VHH antibody having the amino acid sequence of SEQ ID NO: 1,
b) determining the amount of amyloid β peptide 42 oligomers in said biological sample, tissue or brain, and
c) comparing the amount determined in step (b) with a standard, a difference in amount constituting a marker of the presence of said disorder (for instance Alzheimer's disease or Down Syndrome).

As used herein, the term "standard" refers to the amount of amyloid β peptide 42 oligomers in said appropriate biological or in the brain, which has been determined in a large population of subjects not suffering from a disorder mediated by amyloid β peptide oligomers.

In another aspect, the present invention relates to the use of a VHH antibody of camelid binding an epitope located at the C-terminal end of an amyloid β peptide 42 as defined above, preferably the VHH antibody of camelid having the amino acid sequence of SEQ ID NO: 1 for the preparation of a medicament for treating or preventing a disorder mediated by amyloid β peptide oligomers, preferably by amyloid β peptide 42 oligomers.

The term "treating" includes the administration of said VHH antibody to a patient who has said disorder, a symptom of said disorder or a predisposition toward said disorder, with the purpose to cure, heal, alleviate, relieve, alter, remedy, ameliorate, improve, or affect the disorder, the symptoms of the disorder, or the predisposition toward disorder.

The term "preventing" means that the progression of said disorder is reduced and/or eliminated, or that the onset of said disorder is delayed or eliminated

In a preferred embodiment, said medicament is administered to a subject so as to inhibit the formation of amyloid β peptide fibrils, preferably Aβ42 fibrils, and/or to slow down the progression of said disorder.

In a particular embodiment of the present invention, the medicament can be administered to a subject (a mammal or a human) either directly into the brain or by injection, preferably by intravenous, intraperitoneal, intramuscular or subcutaneous injection. Indeed, the Inventors have shown that the said VHH antibody is capable of transmigrate across a blood-brain barrier model.

In another preferred embodiment, the disorder is a neurodegenerative disorder such as Alzheimer's disease or the Down syndrome.

All amyloid fibrils share common features, including a high content of β-sheet in a classical « cross- β » pattern, a fibrillar morphology in electron microscopy, and the ability to bind and alter the spectroscopic properties of hetero-chromatic dyes Congo red and Thioflavin T (Sumner Makin and Serpell, 2005; Westermark, 2005).

Then, the present invention also relates to a method of monitoring the therapeutic effect of a VHH antibody of camelid binding an epitope located at the C-terminal end of an amyloid β peptide 42 as defined above, preferably the VHH antibody of camelid having the amino acid sequence of SEQ ID NO: 1 on the regression of a disorder mediated by amyloid β peptide oligomers in a subject comprising the steps of:
a) contacting *in vitro* or *ex vivo* an appropriate biological sample with a compound binding β-sheet amyloid structures such as amyloid fibrils, preferably Congo Red (sodium salt of benzidinediazo-bis-1-naphtylamine-4-sulfonic acid) or thioflavin T (ThT),
b) determining the amount of β-sheet amyloid structures,
c) comparing the amount so determined with amounts previously obtained for the subject, a decrease in amount constituting a marker of the regression of said disorder.

In addition to the preceding features, the invention further comprises other features which will emerge from the following description, which refers to examples illustrating the present invention, as well as to the appended figures.

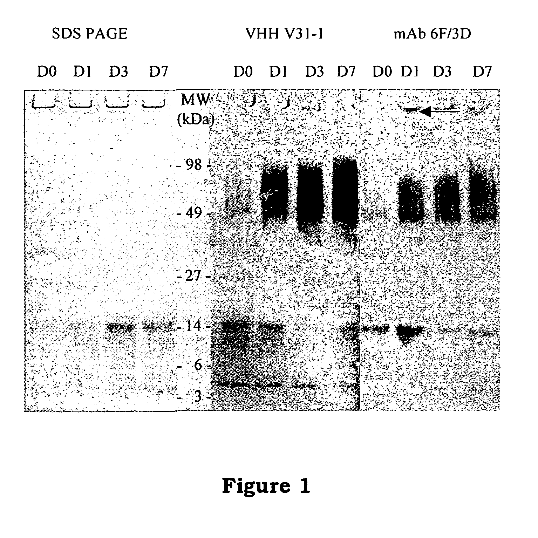

**Figure 1** shows SDS PAGE electrophoresis and immunoblot by V31-1 and mAb 6F/3D. Aβ42 was incubated for several days at 37°C and an aliquot was removed at day 0, 1, 3 and 7. The different fractions were resolved on a 4-12% gel and proteins were transferred onto nitrocellulose membrane. It has to be noted the absence of immunoreactivity of V31-1 for the gel-excluded Aβ reactive material (arrow). Molecular weights are expressed in kDa between the coomassie staining and the immunoblots.

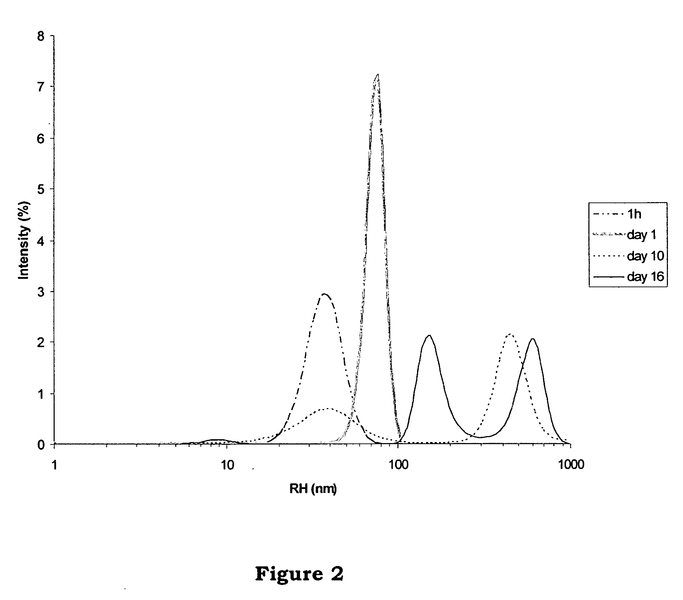

**Figure 2** shows the analysis of Aβ42. size by DLS. Aβ42 was resuspended in water (0.20 µM) and incubated at 37°C for several days. Each R_{H} distribution was normalized to 100 % intensity. Scattering intensity came predominately from polymeric and aggregated Aβ42. Large particles (>1000 nm) were not included in the measurement window. The data are representative of those obtained in each of at least three independent experiments.

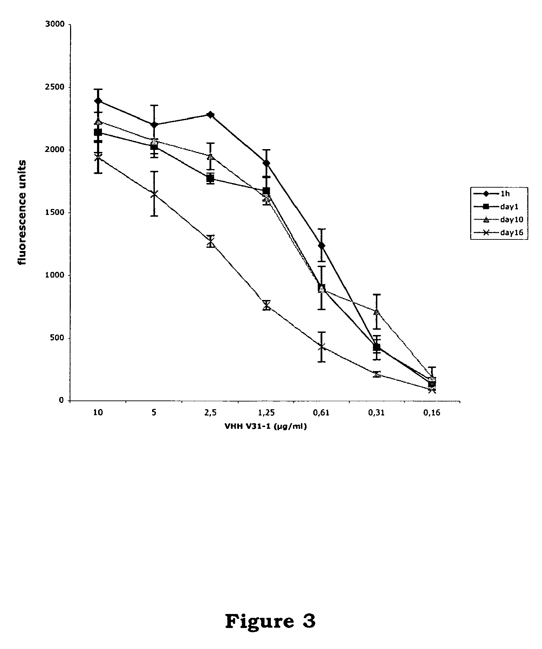

**Figure 3** shows the binding of VHH V31-1 on Aβ42 fractions. Aβ42 were incubated at 37°C for several days and an aliquot was removed at t=lh, at day 1, day 10 and day 16 and stored at -20°C until further use. The coating of fractions was performed at 4°C overnight and the binding of VHH V31-1 at 4°C for 20 min. Two wells were used for each VHH antibody dilution. Results are expressed as means and standard deviations. This experiment is representative of three independent experiments.

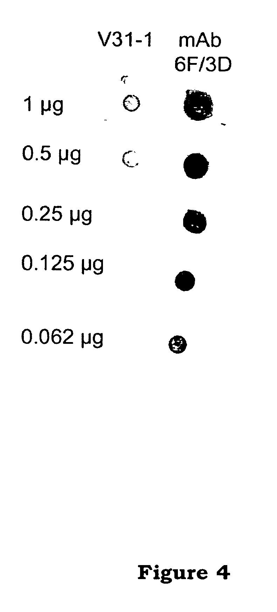

**Figure 4** shows the dot-blot immunoassay of Aβ42 by VHH V31-1 and mAb 6F/3D. 2 µl of serial dilutions of Aβ42 (1 µg, 0.5 µg, 0.25 µg, 0.125 µg, 0.062 µg) were dot-blotted.

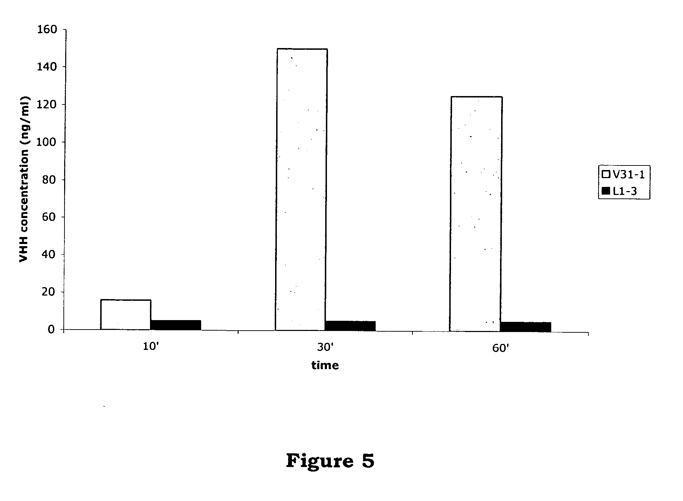

**Figure 5** shows VHH antibody transmigration across *in vitro* blood-brain barrier (BBB). Transport studies were initiated by adding 10-20 µg/ml VHH V31-1 or L1-3 antibody to apical compartment (upper chamber) and the amount of VHH antibodies was determined in the lower chamber at 10 min, 30 min and 60 min.

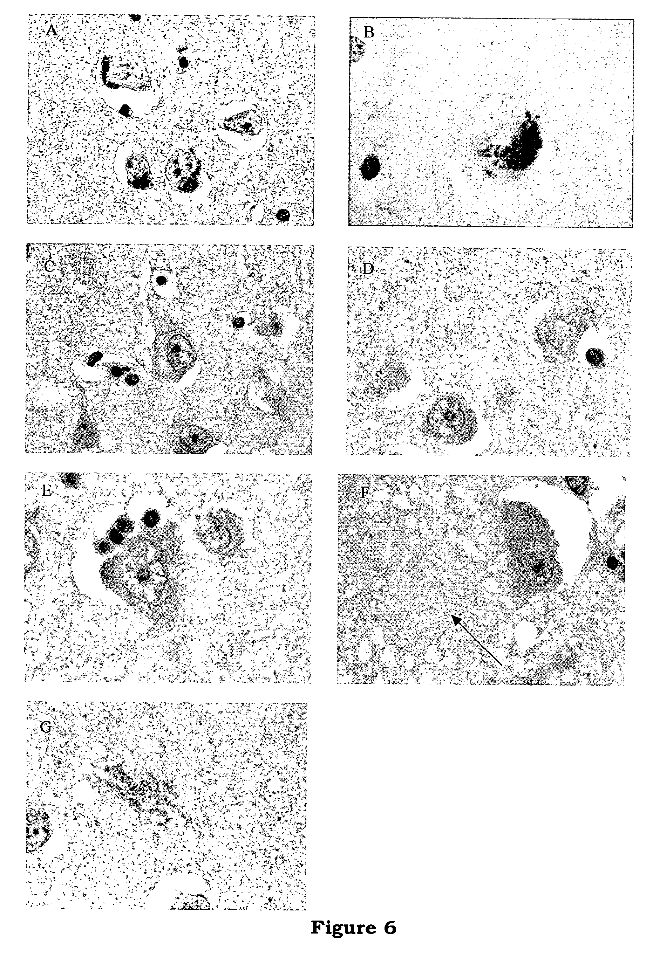

**Figure 6** shows the intraneuronal Aβ42 peptide immunoreactivity of AD patient by various VHHs. Representative photomicrographs of brain tissue stained with VHH V31-1 (**A** and **B**), VHH L1-3 **(C)** and VHH 61-3 **(D).** When VHH V31-1 is pre-incubated with Aβ42 prior use, only faint neuronal staining can be seen **(E).** The photomicrograph **F** demonstrates both intraneuronal Aβ42 immunoreactivity and the lack of extraneuronal diffuse plaque staining by VHH V31-1 (plaques are indicating by an arrow). The synapses are labelled by VHH V31-1 (**G**).

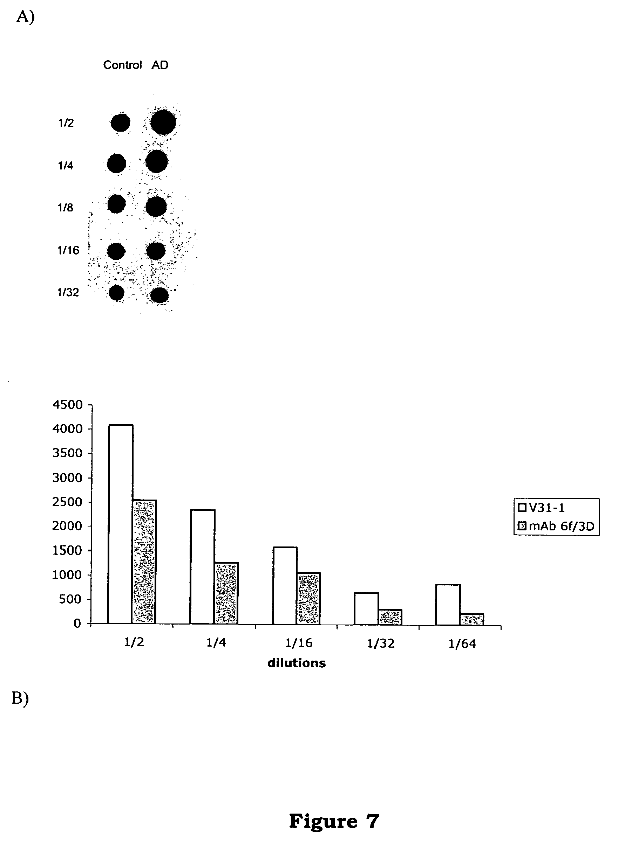

**Figure 7** shows Aβ42 immunoreactivity by dot-blot immunoassay of the formic acid fractions of control and AD patient. 2 µl of serial dilutions (1/2, 1/4, 1/8, 1/16, 1/32, 1/64, 1/128) were dot-blotted. VHH V31-1 (**A**) and mAb 6F/3D were used. Aβ42immunoreactivity was determined by densitometry for all of the dilutions. Aβ42 quantities in relative absorbance units (R.A.U.), corresponding to the difference of signal between the AD and control patient, are expressed as a function of sample dilution (**B**).

The following examples illustrate the invention but in no way limit it.

### EXAMPLE 1: Materials and methods

### Materials

Aβ42 (SEQ ID NO: 2) and the different Aβ42 fragments (1-11, 10-20, 15-25, 22-35, 29-40 and 33-42 fragments of SEQ ID NO: 2, respectively SEQ ID NO: 5, 6, 7, 8, 9 and 10) used were purchased from Bachem. The monoclonal antibody (mAb) 6F/3D anti-Ab 8-17 (Dako) recognizes synthetic amyloid peptides by dot- and western-blotting. It also specifically stains all type of amyloid deposits in AD brains.

### Subjects

Human cortical brain tissue was obtained from the Hôpital Pitié-La Salpetrière, Paris, France. Postmortem brain tissue was examined from representative neurologically normal controls and AD patient (staged Braak-VI, according to Braak and Braak, 1991).

### VHH antibodies and expression thereof in a pET system

VHH V31-1 (SEQ ID NO: 1)
VHH L1-3 (SEQ ID NO: 3)
VHH 61-3 (SEQ ID NO: 12)

The coding sequences of VHH V31-1, VHH L1-3 and VHH 61-3 antibodies inserted in vector pHEN1, described in International Application No. WO 2004/044204, were subcloned in vector pET22 using the *Nco*I and *Not*I restriction sites according to the manufacturer's instructions (Novagen, Darmstadt, Germany). Transformed *E. coli* BL 21 (DE3) cells expressed VHH antibodies in the periplasm after induction by IPTG 1 mM for 3 hours at 20°C. Periplasmic extracts were obtained by spheroplasting cells, suspended in 50 mM sodium phosphate buffer pH 8 containing 20% sucrose and 1 mM EDTA, and hydrolysing the peptidoglycan with 5 mg/ml lysozyme for 20 min at 4°C, in the presence of protease inhibitors (Complete™, Boehringer Mannheim, Germany). The suspension was then centrifuged 2 min at 10,000 rpm. The supernatant corresponding to the periplasmic extract was kept at 4°C. Purified VHH antibodies were obtained by IMAC using a chelating agarose column charged with Ni²⁺ (Superflow Ni-NTA, Qiagen Ltd, UK) according to manufacturer's instructions. The protein content was measured using the Bradford reagent. The purity of the final preparation was evaluated by SDS-PAGE with Coomassie staining and by Western blot.

### ELISA

A modified version of a standard ELISA was used to test for the presence of VHH antibodies in culture supernatants. Microtiter plates (Nunc, Denmark) were coated by incubation overnight at 4°C with 1 µg/ml of antigen diluted in PBS. Plates were washed four times with buffer A (0.1% Tween 20 in PBS), and VHHs were diluted in buffer B (0.5% gelatin in buffer A). The plates were incubated for 2 hours at 37°C and washed again, before adding a horseradish peroxidase-labeled rabbit anti-*c*-*myc* (A14) (Santa Cruz, Ca, USA) or with a rabbit anti-His tag antibody (Santa Cruz, Ca, USA). Then, the plates were washed with buffer A, and freshly prepared 0.2 % orthophenylenediamine (Dakopatts A/S, Glostrup, Denmark), 0.03% H₂O₂ in 0.1 M citrate buffer, pH 5.2, were added to each well. The peroxidase reaction was stopped by adding 3 M HCl, and the optical density was measured at 490 nm.

### Determination of dissociation constants by ELISA

Binding affinity of VHH antibodies was determined as described in Friguet *et al.,* (1985). Various concentrations of Aβ peptides were incubated in solution overnight at 4°C with a known quantity of VHH antibody until equilibrium was reached. The VHH antibody concentration used was determined by preliminary ELISA calibrations. Each mixture (100 µl) was transferred to a well of a microtiter plate previously coated with antigen and was incubated for 20 min at 4°C. The plates were washed with buffer A and free VHH antibodies were detected by adding β-galactosidase-conjugated goat anti-rabbit Igs (Biosys, Compiègne, France) and 4-methylumbelliferyl β-D galactoside (Sigma). Fluorescence was read (Fluoroskan, Labsystem, Finland) at 460 nm, after excitation at 355 nm. *K*_{D} was estimated from the slope of the regression curve obtained by plotting the reciprocal of the fraction of bound antibody versus the reciprocal of the molar concentration of antigen.

### Western Blot

Aβ peptides were suspended in PBS. To an aliquot (5µl), an equal volume of gel loading buffer was added and then treated at 100°C for 5min. Following separation by polyacrylamide gel electrophoresis (PAGE) using NuPAGE Novex 4-12% Bis-tris gel (Invitrogen), semi-dry transfer onto Hybond-C (Amersham) and western blotting were carried out using the Xcell II blot module (Invitrogen). Prior to the immunochemical reaction, membranes were blocked in a 4% skimmed milk solution. Immunoblotting of membranes was accomplished with either VHH antibody or mAb, and respectively revealed by peroxidase-labeled rabbit anti-c-myc (A14) antibodies (Santa Cruz, Ca, USA), or rabbit anti-His tag (Santa Cruz, Ca, USA) followed by peroxidase labeled goat anti-rabbit immunoglobulins. Finally, peroxidase activity was visualized using a chemiluminescent kit (Amersham).

### Dot-blot

Frozen brain samples were homogenized (1:10 (w/v)) in the Laemmli sample buffer containing 0,25% (W/v) dithiothreitol (DTT) and heat-treated for 10 min. Each sample was then centrifuged at 100,000 g for 60 min. Resulting pellets were resuspended in 500 µl of 100% formic acid and left under agitation at room temperature for 3 hours. Fractions were then centrifuged for 1 hr at 14000xg (Permanne *et al.,* 1995). The supernatants were removed and 2 µl of a serial dilution (from 1/2 to 1/164) were dot-blotted onto Hybond-C nitrocellulose membranes (Amersham). Membranes were blocked and processed for immunorevelation as described for the western blot. Dot-blots were digitized on a Gel-doc (Biorad) at a resolution of 72 dots/inch and saved as 8-bit gray scale PICT files (256 shades of gray). Intensities of immunoreactivity were calculated for each dot with Quantity one software (Biorad).

### Preparation of Aβ42 monomers and protofibrils

Samples of 1 mg of Aβ42 powder were dissolved in 500 µl of hexafluoroisopropanol (Sigma), gently stirred at 4°C for 7 days, sonicated for 10min using a Branson ultrasonic bath sonicator and then centrifuged for 10 min at 16000xg. Aliquots containing 50 µg of peptides each were lyophilised and stored at -20°C. Aliquots were dissolved in double distilled water or in PBS pH=7.4 and incubated at 37°C until use.

### Dynamic Light Scattering (DLS)

DLS (also termed quasi-elastic light scattering) was used to measure the average diffusion coefficient distribution of Aβ particles of the particles. Each particle present in the sample is characterized by its hydrodynamic radius (R_{H}), corresponding to the radius of a sphere with a diffusion coefficient equal to that measured. R_{H} measurements were made at 25°C with a DynaPro MS800 instrument (Protein solution-Wyatt) equipped with a gallium aluminium arsenide 825 nm laser. An aliquot of Aβ42 peptide was diluted to 0.20 µM in double-distilled water and incubated at 37°C. Samples (110 µl) were placed directly into a 3 mm optical pathlength quartz cuvette (Hellma), and the total light scattering intensity at a 90° angle was collected using a 10-s averaging acquisition time. Particle translational diffusion coefficients (D_{T}) were calculated from autocorrelated light intensity data (usually 30-40 points) and converted to R_{H} with the Stokes-Einstein equation. A distribution plot of intensity versus R_{H} was calculated using the Sedfit 9.3 analysis software (www.analyticalultracentrifugation.com), and intensity-weighted mean R_{H} values were obtained from each subpeak.

### Thioflavin T (ThT) fluorescence assay

Fluorescence emission of ThT is shifted when it binds to β-sheet aggregate structures such as amyloid fibrils (LeVine, 1993). An aliquot of Aβ42 peptide was diluted to 20 µM in double-distilled water or in PBS pH 7.4 and incubated at 37°C. Aβ42 aggregation was measured by periodically removing 30 µl aliquots from the incubation samples and adding them to 2 ml of 5 µM ThT solution (50 mM phosphate buffer, pH=6.5). Fluorescence intensity was monitored at an excitation wavelength of 450 nm and an emission wavelength of 482 nm on a spectrofluorometer using 1 cm light-path quartz cuvettes with both excitation and emission bandwidths of 5 nm. Readings were the results of an average of three values after substracting the fluorescence contribution of free ThT. Each experiment was performed in duplicate.

### Statistical analysis

A two-tailed Student's t-test was used for comparison of the fibrillogenesis of Aβ42 in the presence or absence of antibody fragments. P values < 0.05 were considered as statistically different.

### Immunocytochemistry

Immunostaining of brain tissue was performed on 7 µM thick paraffin sections. Sections were de-paraffinized in xylene, rehydrated through ethanol (100%, 96 %, and 90%) and finally brought to water. They were incubated in 90% formic acid, washed again in water, quenched for endogenous peroxidase with 3% hydrogen peroxyde and 20% methanol, and finally washed in water. Non-specific binding was blocked by incubating the sections for 10 minutes in 2% bovine serum albumin in TBS + 0,5% Tween. Appropriate dilutions of primary antibodies were then applied overnight in a humidified chamber at room temperature (typically 1 µg/ml for VHH, and 1:200 for 6F/3D mAb). Slides were washed with TBS-Tween and incubated with secondary antibodies (rabbit anti-His Tag or biotinylated anti-mouse immunoglobulins) in TBS-Tween at room temperature for 2 hours. Slides were then incubated with either peroxidase goat anti-rabbit immunoglobulins or streptavidine-peroxidase fusions, and developped with diaminobenzidine (DAB) for 2 minutes. After washing with TBS-Tween, slides were counter-stained with hematoxylin and eosin.

### EXAMPLE 2: Demonstration of the recognition of oligomeric forms of Aβ42 by VHH V31-1

### 1. Characterization of the epitopes recognized by VHH V31-1

International Application No. WO 2004/044204 discloses that VHH V31-1 recognizes the carboxy terminal end of Aβ42. To further characterize the epitope recognized by VHH V31-1, the K_{D} was determined for peptides corresponding to different Aβ fragments (1-11, 10-20, 15-25, 22-35, 29-40 and 33-42). VHH V31-1 did not recognize the fragments 1-10, 10-20, 15-25 and 22-35. The K_{D} of VHH V31-1 for Aβ42, Aβ 29-40 and Aβ 33-42 was in the same order of magnitude, respectively 2(±0.8).10⁻⁸ M for Aβ 29-40 and 2.10⁻⁸ M for Aβ 33-42, suggesting that VHH V31-1 recognizes an epitope located at the C-terminal end of Aβ42.

### 2. VHH V31-1 recognizes preferentially the oligomeric form of Aβ42

To further characterize VHH V31-1, its binding was tested by immunoblotting on Aβ. Aβ42 was incubated for several days at 37°C and an aliquot was removed at days 0, 1, 3 and 7. Standard Aβ42 preparation, *i.e.,* « aging » by incubation of high concentrations of Aβ42 at 37°C for several days, lead to mixed fractions. After SDS denaturation, such preparations showed not only gel-excluded Aβ-reactive material *(i.e.,* SDS-insoluble fibrils), but also Aβ42 monomers and oligomers (Hartley *et al.,* 1999). VHH V31-1 labelled the 4 kDa band corresponding to monomer, the 8-16 kDa bands corresponding to dimers, trimers and tetramers, and the 50-80 KDa bands corresponding to 12-mers, but not the additional band at the very top of the gel, suggesting that V31-1 recognizes specifically Aβ42 in its oligomeric form, but not its fibrillar form (Figure 1). In contrast, mAb 6F/3D labelled all the different bands including the gel-excluded fraction.

DLS was then used to study the evolution of the size distribution of Aβ42 particles along the oligomerization process. Aβ42 peptide was dissolved in hexafluoroisopropanol (HFIP), sonicated and then centrifuged to completely remove associated peptides. Lyophilized aliquots were kept at -20°C, resuspended in double-distilled water and analyzed by DLS at different incubation times. At T=1h, the size distribution for Aβ42 showed a single peak with a weight-average hydrodynamic radius (R_{H}) of approximately 40 nm (Figure 2). At day 1, a single peak centred around 80 nm was observed. At day 10, two peaks were observed: one centred around 40 nm and the other around 400-500 nm. At day 20, two peaks were also observed: one centred around 150 nm and the second around 600-800 nm. It is important to note that because the particle light scattering intensity is proportional to the volume filled by a molecule (I_{D} = f(R_{H}³, C, 1/λ⁴)), the actual abundance of large particles is significantly lower than suggested by the intensity distributions. Bitan et al previously analysed the oligomer size distribution of Aβ42 by DLS (Bitan *et al.,* 2003). Two peaks were observed, one around 10-20 nm, corresponding to pentamer/hexamer units, and the other around 60 nm, corresponding to protofibrils, formed by further self-association of these oligomers. The 40 nm peak that was observed could be a weight-average of the two peaks reported at 10-20 nm and 60 nm

The kinetics of fibril formation was monitored using a ThT assay (Table I below). ThT is a fluorescent dye that specifically binds to fibrillar structures. Aβ42 was resuspended in water and aliquots were periodically removed and added to a ThT solution. A time-dependent increase was observed, suggesting that progressive formation of amyloid fibrils was taking place.

**Table I: Kinetics of Aβ42 fibrillogenesis. ThT binding shows an increase at day 1 and 5. Background fluorescence (0.7 10⁵ cpm) was subtracted for all measurements.**

| Incubation time | Fluorescence (10⁵ cpm) | Relative response |
|---|---|---|
| T=1h | 1.19 | 1.0 |
| T= day 1 | 4.86 | 4.1 |
| T= day 6 | 9.90 | 8.3 |

The binding of V31-1 to the different fractions of Aβ42 was analysed in ELISA (Figure 3). The binding of VHH V31-1 to Aβ42 was significantly higher for the 1h-fractions than for the day 16 fractions.

Dot-blot immunoassays were performed on Aβ42 incubated for more than 20 days at 37°C, composed mostly of fibrils. A strong Aβ42 immunoreactivity was obtained with mAb 6F/3D. Conversely, Aβ42 immunoreactivity was weak with VHH V31-1 (Figure 4), thus confirming that it preferentially binds to an oligomeric form of Aβ and not to fibrillar structures.

### 3. VHH V31-1 recognizes intraneuronal Aβ42 but not amyloid plaques

The distribution of VHH-specific immunoreactivity in human AD brains was examined. Stained AD brain tissue slices revealed significant intraneuronal immunoreactivity for VHH V31-1, while that of VHH 61-3 was very faint and that of VHH L1-3 undetectable (Figure 6A-D). Normal brain tissues were not labelled with VHHs. The granules were located in the perinuclear region of the cell body. The cells containing the granules were identified as neuronal by their shape. Endothelial cells never contained granules. The specificity of this labelling was confirmed by pre-incubating VHH V31-1 with Aβ42 (Fig 6E). VHH failed to detect amyloid plaques but detected intracellular granules of neurons surrounding such plaques (Fig 6G). Synapses were also labelled with V31-1 (Fig 6F).

To confirm the immunoreactivity of VHH V31-1 on brain tissues, dot-blot immunoassays were performed on formic acid fractions obtained from an AD patient and from a control. A strong Aβ42 immunoreactivity was obtained in the AD patient fraction. Conversely, Aβ42 immunoreactivity was weak for the control patient (Figure 7A). To compare the results obtained for VHH V31-1 and mAb 6F/3D, Aβ42 immunoreactivity was determined by densitometry for all the dilutions. Aβ42 quantities corresponding to the difference of signal between the AD and control patients, were expressed as a function of sample dilution. The results obtained with VHH V31-1 and mAb 6F/3D were similar (Figure 7B). Thus the present assay confirmed that VHH V31-1 was able to detect Aβ42 in brain tissues.

### 4. VHH V31-1 inhibits the formation of fibrils

To investigate the effects of the VHH antibodies on fibril formation, conditions were selected (lyophilized Aβ42 is resuspended in water then diluted in PBS at the working concentration and sonicated) under which both Aβ42 is able to aggregate *in vitro* and the VHH antibody can bind tightly to Aβ42. Similar experiments were performed with an irrelevant VHH antibody (L35; SEQ ID NO: 4; described in International Application No. WO 2004/044204) as a negative control. A striking feature of the VHH antibodies is their capacity to withstand prolonged incubation at 37°C (Arbabi Ghahroudi *et al.,* 1997).

In the absence of VHH V31-1, the DLS data showed that Aβ42 readily aggregated with the formation of 2 peaks at day 1, one around 30 nm and the other around 100-150 nm. Then as early as day 2, a new peak centred around 300 nm could be detected, which then evolved to 600-700 nm at day 7 (Table II).

**Table II: Effects of VHH on aggregation of Aβ42. Time course of the aggregation of Aβ42 (0.20 µM) in the absence and presence of equimolar concentrations (0.20 µM) of VHH V31-1 and VHH L35 monitored by light scattering. Large particles (>1000 nm) were not included in the measurement window.**

| | Aβ42 | | Aβ42 + V31-1 | | Aβ42+L35 | | |
|---|---|---|---|---|---|---|---|
| 3h | | | | | | | |
| R_{H} (nm) | | 88 | | 97 | | 109 | |
| 5h | | | | | | | |
| R_{H} (nm) | | 95 | | 84 | | 116 | |
| 24h | | | | | | | |
| R_{H} (nm) | 24 | 125 | 10 | 101 | 14 | 128 | |
| day 2 | | | | | | | |
| R_{H} (nm) | 35 | 329 | 9 | 122 | 44 | 250 | |
| day 3 | | | | | | | |
| R_{H} (nm) | 36 | 208 | 25 | 256 | 33 | 232 | |
| day 4 | | | | | | | |
| R_{H} (nm) | 60 | 498 | 12 | 157 | | 135 | 663 |
| day 7 | | | | | | | |
| R_{H} (nm) | 41 | 142 665 | 25 | 151 | | 107 | 701 |

The aggregation of Aβ42 therefore appears to be similar when it is diluted in water (Figure 2) or in PBS (Table II). When Aβ42 is co-incubated with an equimolar concentration of VHH L35, similar pattern of aggregation was observed with the formation of 2 main peaks around 100-150 nm and 600-700nm. Interestingly, the kinetic of fibril formation appeared to be faster when Aβ42 was incubated with VHH L35 than when Aβ42 was alone, as the 600-800 nm peak appeared as early as day 4. When Aβ42 was co-incubated with an equimolar concentration of VHH V31-1, two peaks were observed, one around 10-25 nm and one centred around 150 -200 nm. Noticeably, the 600-800 nm peak could never be detected for this complex, suggesting that high molecular weight aggregates could not form in the presence of VHH V31-1. The average light scattering intensity of VHH antibodies alone is much lower than the intensity of the VHH antibodies incubated with Aβ42. The absence of formation of fibrils was also confirmed by the ThT assay: fluorescence actually significantly decreased when Aβ42 was co-incubated with an equimolar concentration of VHH V31-1, while it increased when Aβ42 was alone (Table III).

**Table III: Time course of the fibrillogenesis of Aβ42 in the absence and presence of antibody fragments. The kinetics of Aβ42 (20 µM) fibril formation was monitored by ThT fluorescence in the absence and presence of equimolar concentrations (20µM) of VHH V31-1 and VHH L35. The samples were incubated at 37°C and 30 µl of the samples were removed periodically and added to 2 ml of 5µM ThT. P values resulting from paired t-test analysis: * P < 0.05, ** P<0.01, *** P<0.001 (n=4). This experiment is representative of two independent experiments.**

| | Fluorescence (10⁵ cpm) | | |
|---|---|---|---|
| Incubation time | Aβ42 | Aβ42 + VHH V31-1 | Aβ42 + VHH L35 |
| Day 0 | 2,3 ± 0.15 | 1.86 ± 0.15 | 1.66 ± 0.18 |
| Day 1 | 4,47 ± 0.63 | 2.85 ± 0.48* | 2.8 ± 0.53* |
| Day 5 | 3,33 ± 0,14 | 1.3 ± 0.14*** | 3.83 ± 0.36 |
| Day 10 | 4,4 ± 0.46 | 1.7 ± 0.14*** | 3.07 ± 0.33* |
| Day 15 | 7.65 ± 2.18 | 2.06 ± 0.08** | 4.21 ± 1.3 |

No significant decrease of fluorescence was observed when Aβ42 is co-incubated with an equimolar concentration of VHH L35.

Yan and Wang (2006) have recently shown that the C terminus of Aβ42 is more rigid than that of Aβ40 and is likely preordered for its β-conformation in fibrils and oligomers, therefore acting as an internal seed for aggregation. This notion is supported by recent bioinformatic studies of Aβ42 showing that the C-terminal six-residues of Aβ42 have the best match for the NNQQNY conformation in the crystal structure of fibrils (Thompson *et al.,* 2006; Nelson *et al.,* 2005). It can be postulated that VHH V31-1 may recognize this seed in oligomers and preclude the formation of aggregates.

### EXAMPLE 3: In vitro VHH antibody transmigration across hCMEC/D3 Methods

Immortalized human brain endothelial cells hCMEC/D3 have been previously described in detail in Weksler *et al.* (2005). Cell viability in the presence of VHH antibodies was assessed by MTT assay as described in Weksler *et al.* (2005).

The permeability of hCMEC/D3 cell monolayers to VHH antibodies was measured on transwell polycarbonate insert filters (pore size 3 µm, Coming, Brumath, France) as described in Weksler *et al.* (2005). hCMEC/D3 cells were seeded on the filters at a confluent density of 2x10⁵ cells/cm² in EGM-2 medium.

Transport studies were performed 3 days post-seeding as described in Weksler *et al.* (2005). Experiments were initiated by adding VHH antibodies to the upper chamber containing either collagen, coated inserts without cells, hCMEC/D3 cells or hCMEC/D3 cells pre-exposed to various pharmacological modulators for 30 min. Transport studies were conducted at 37°C. The lower chamber was sampled at various time intervals (10, 30 and 60 min) and the presence of VHH antibodies was determined by ELISA and Western Blot (see below).

### Results

Figure 5 shows that there is a transcytosis of functional VHH V31-1 while there is no passage of VHH L1-3 across hCMEC/D3. This passage was time-dependant and reached a maximum at 30 min. At 60 min about 1% of VHH V31-1 antibody was present in the lower chamber. This result shows that VHH V31-1 is able to transmigrate across the blood-brain barrier.

### REFERENCES

- Arbabi Ghahroudi M. et al., FEBS Letters, 1997, 414, 521-6.
- Balbach J. J. et al., Biochemistry, 2000, 39, 13748-59.
- Bard F. et al., Nature Medicine, 2000, 6, 916-9.
- Bitan G. et al., Proc Natl Acad Sci U S A, 2003, 100, 330-5.
- Cleary J. P. et al., Nat Neurosc, 2005, 8, 79-84.
- Desmyter A. et al., Nature Structural Biology, 1996, 3, 803-11.
- Desmyter A. et al., Journal of Biological Chemistry, 2002, 277, 23645-23650.
- Friguet B. et al., Journal of Immunological Methods, 1985, 77, 305-319.
- Gong Y. et al., Proc Natl Acad Sci U S A, 2003, 100, 10417-22.
- Halverson K. et al., Biochemistry, 1990, 29, 2639-2644.
- Hartley D. M. et al., J Neurosci, 1999, 19, 8876-84.
- Hilbich C. et al., J Mol Biol, 1991, 218, 149-63.
- Jarrett J. T. et al., Biochemistry, 1993, 32, 4693-7.
- Kayed R. et al., Science, 2003, 300, 486-9.
- Klein W. L. et al., Trends in Neurosciences, 2001, 24, 219-224.
- Kodali S. et al., Current Opinion in Structural Biology, 2007, 17, 48-57.
- Kuo Y. M. et al., J Biol Chem, 1996, 271, 4077-81.
- Lacor P. N et al., J Neurosci, 2004, 24, 10191-200.
- Lambert M. P. et al., Proc Natl Acad Sci U S A, 1998, 95, 6448-53.
- Lambert M. P. et al., J Neurochem, 2001, 79, 595-605.
- Lambert M. P. et al., J Neurochem, 2007, 100, 23-35.
- Lauwereys M. et al., Embo J, 1998, 17, 3512-20.
- Lee E. B. et al., J Biol Chem, 2006, 281, 4292-9.
- Legleiter J. et al., J Mol Biol, 2004, 335, 997-1006.
- LeVine H., Protein Sci, 1993, 2, 404-10.
- Liu R. et al., Biochemistry, 2004, 43, 6959-67.
- Lomakin A. et al., Proc Natl Acad Sci U S A, 1997, 94, 7942-7.
- Ma B. and Nussinov R., Proc Natl Acad Sci U S A, 2002, 99, 14126-31.
- Muyldermans S. et al., Trends in Biochemical Sciences, 2001, 26, 230-235.
- Nelson R. et al., Nature, 2005, 435, 773-8.
- Nguyen VK. et al., Adv. Immunol., 2001, 79, 261-96
- Permanne B. et al., Brain Res, 1995, 685, 154-62.
- Solomon B., Current Medicinal Chemistry, 2002, 9, 1737-1749.
- Sumner Makin O. and Serpell L.C., FEBS Letters, 2005, 205, 5950-5961.
- Thompson M. J. et al., Proc Natl Acad Sci U S A, 2006, 103, 4074-8.
- Townsend M. et al., J Physiol, 2006, 572, 477-92.
- Transue T. R. et al., Proteins, 1998, 32, 515-22.
- Walsh D. M. et al., Nature, 2002, 416, 535-9.
- Wang H. W. et al., Brain Res, 2002, 924, 133-40.
- Wang Q.et al., J Neurosci, 2004, 24, 3370-8.
- Weksler B.B. et al., The FASEB Journal, 2005, 19, 1872-1874
- Westermark P., FEBS Letters, 2005, 272, 5942-5949.
- Yan Y. and Wang C., J Mol Biol, 2006, 364, 853-62.

## Claims

1. Use of a VHH antibody of camelid binding an epitope located at the C-terminal end of the amyloid β peptide 42 for detecting *in vitro* in an appropriate biological sample, an oligomeric form of the amyloid β peptide 42 having the amino acid sequence of SEQ ID NO: 2.

2. The use according to claim 1, **characterized in that** said epitope comprises, is comprised or consists of a peptide selected from the group consisting of the peptide Aβ 29-40 having the amino acid sequence of SEQ ID NO: 9, the peptide Aβ 33-42 having the amino acid sequence of SEQ ID NO: 10 and the peptide Aβ 35-42 having the amino acid sequence of SEQ ID NO: 11.

3. The use according to claim 1 or claim 2, **characterized in that** said VHH antibody has the amino acid sequence of SEQ ID NO: 1.

4. The use according to any of claims 1 to 3, **characterized in that** the oligomeric form of said amyloid β peptide 42 is selected from the group consisting of a 2-mer, 3-mer, 4-mer, 5-mer, 6-mer, 7-mer, 8-mer, 9-mer, 10-mer, 11-mer, 12-mer, 13-mer, 14-mer, 15-mer, 16-mer, 17-mer, 18-mer, 19-mer, 20-mer, 21-mer, 22-mer, 23-mer, 24-mer, more preferably a dimer, trimer, tetramer or dodecamer.

5. The use according to any one of claims 1 to 4, **characterized in that** the presence of an oligomeric form of the amyloid β peptide 42 in said biological sample is a prognostic marker of appearance of a disorder mediated by amyloid β peptide oligomers.

6. The use according to any of claims 1 to 5, **characterized in that** said disorder is Alzheimer's disease or the Down syndrome.

7. An *in vitro* method for determining an early-stage of a disorder mediated by amyloid β peptide oligomers, **characterized in that** it comprises a step of detecting in a subject an oligomeric form of the amyloid β peptide 42 by contacting an appropriate biological sample or tissue with a VHH antibody as defined in any of claims 1 to 3.

8. The method according to claim 7 or claim 8, **characterized in that** said disorder is Alzheimer's disease or the Down syndrome.

9. A method of diagnostic of a disorder mediated by amyloid β peptide oligomers in a subject comprising the steps of:
a) contacting *in vitro* or *ex vivo* an appropriate biological sample with a VHH antibody as defined in any of claims 1 to 3, and
b) detecting the binding of said antibody to said biological sample, a binding constituting a marker of the presence of said disorder.

10. The method according to claim 9, **characterized in that** said disorder is Alzheimer's disease or the Down syndrome.

11. A method of diagnostic of a disorder mediated by amyloid β peptide oligomers in a subject comprising the steps of:
a) contacting *in vitro* or *ex vivo* an appropriate biological sample with a VHH antibody as defined in any of claims 1 to 3,
b) determining the amount of amyloid β peptide 42 oligomers in said biological sample, and
c) comparing the amount determined in step (b) with a standard, a difference in amount constituting a marker of the presence of said disorder.

12. The method according to claim 11, **characterized in that** said disorder is Alzheimer's disease or the Down syndrome.

13. The method according to claims 7, 9 or 11 **characterized in that** the oligomeric form of said amyloid β peptide 42 is selected from the group consisting of a 2-mer, 3-mer, 4-mer, 5-mer, 6-mer, 7-mer, 8-mer, 9-mer, 10-mer, 11-mer, 12-mer, 13-mer, 14-mer, 15-mer, 16-mer, 17-mer, 18-mer, 19-mer, 20-mer, 21-mer, 22-mer, 23-mer, 24-mer, more preferably a dimer, trimer, tetramer or dodecamer.

14. Use of a VHH antibody as defined in any of claims 1 to 3 for the preparation of a medicament for treating or preventing a disorder mediated by amyloid β peptide oligomers, preferably amyloid β peptide 42 oligomers.

15. The use according to claim 14, **characterized in that** said medicament is administered to a subject so as to inhibit the formation of amyloid β peptide fibrils.

16. The use according to claim 14 or claim 15, **characterized in that** said medicament is administered to a subject so as to slow down the progression of said disorder.

17. The use according to any of claims 14 to 16, **characterized in that** said disorder is a neurodegenerative disorder.

18. The use according to claim 17, **characterized in that** said neurodegenerative disorder is Alzheimer's disease or the Down syndrome.

19. A method of monitoring the therapeutic effect of a VHH antibody as defined in any of claims 1 to 3 on the regression of a disorder mediated by amyloid β peptide oligomers in a subject comprising the steps of:
a) contacting *in vitro* or *ex vivo* an appropriate biological sample with a compound binding β-sheet amyloid structures such as amyloid fibrils,
b) determining the amount of β-sheet amyloid structures,
c) comparing the amount so determined with amounts previously obtained for the subject, a decrease in amount constituting a marker of the regression of said disorder.

20. The method according to claim 19, **characterized in that** said disorder is Alzheimer's disease or the Down syndrome.
